(19) Europäisches Patentamt / European Patent Office / Office européen des brevets

(11) **EP 2 226 630 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
***G01N 27/416*** (2006.01)

(21) Anmeldenummer: **09154512.9**

(22) Anmeldetag: **06.03.2009**

(54) **Verfahren zur Bestimmung eines Zustandsindikators eines Wasser-Analysegerätes**

Method for determining a status indicator of a water analysis device

Procédé de détermination d'un indicateur d'état d'un appareil d'analyse de l'eau

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(43) Veröffentlichungstag der Anmeldung:
**08.09.2010 Patentblatt 2010/36**

(73) Patentinhaber: **Hach Lange GmbH**
**14163 Berlin (DE)**

(72) Erfinder:
• **Streppel, Toon**
**6961, AG Eerbeek (NL)**
• **Seehaus, Torsten**
**40221, Düsseldorf (DE)**
• **Schmitz, Ulrich**
**47647, Kerken (DE)**
• **Battefeld, Manfred**
**40211, Düsseldorf (DE)**
• **Kussmann, Michael**
**40476, Düsseldorf (DE)**
• **Haeck, Michael**
**51429, Bergisch Gladbach (DE)**
• **Thomas, Frank**
**42657, Solingen (NL)**

(74) Vertreter: **Ter Smitten, Hans**
**Patentanwälte ter Smitten**
**Burgunder Strasse 29**
**40549 Düsseldorf (DE)**

(56) Entgegenhaltungen:
**WO-A-2004/025223 DE-A1- 10 209 318**
**DE-A1-102004 063 468**

**Beschreibung**

Die Erfindung bezieht sich auf ein Verfahren zur Bestimmung eines Zustandsindikators eines Wasser-Analysegerätes.

Wasser-Analysegeräte dienen der qualitativen und quantitativen Bestimmung eines oder mehrerer Analyte in Wasser, beispielsweise in Abwasser oder Trinkwasser. Wasser-Analysegeräte können als sogenannte Laborgeräte für Einzelmessungen oder als Prozessgeräte für quasi-kontinuierliche Messungen ausgebildet sein.

Wasser-Analysegeräte nach dem Stand der Technik liefern keine oder nur unzureichende Informationen über den technischen Zustand des Analysegerätes, so dass es nicht ohne weiteres möglich ist, den Gesundheitszustand des Analysegerätes oder die Vertrauenswürdigkeit der von dem Analysegerät gelieferten Messwerte zu beurteilen.

Aufgabe der Erfindung ist es demgegenüber, ein Wasser-Analysegerät zu schaffen, das in Form eines Zustandsindikators eine Aussage über den Gesamtzustand des Analysegerätes zur Verfügung stellt. Ein solches Analysegerät mit Funktionsüberwachung ist bekannt aus DE 10 2004 063 468.

Diese Aufgabe wird erfindungsgemäß gelöst mit einem Verfahren mit den Merkmalen des Patentanspruches 1.

Gemäß dem erfindungsgemäßen Verfahren sind zur Ermittlung eines Analysegerät-Zustandsindikators folgende Verfahrensschritte vorgesehen:

Ermittlung jeweils eines Parameterwertes für mindestens zwei verschiedene technische Parameter des Wasser-Analysegerätes,

Ermittlung eines Abweichungswertes des Parameterwertes in Bezug auf einen zugeordneten Parameter-Referenzwert für jeweils alle Parameter, Ermittlung jeweils eines Abweichungsrelevanz-Wertes aus dem Abweichungswert mit Hilfe einer parameterspezifischen Abweichungsrelevanz- Funktion für jeweils alle Parameter, wobei sich die Funktionen voneinander unterscheiden, und

Errechnen des Zustandsindikators aus allen ermittelten Abweichungsrelevanz-Werten mit einer Indikatorfunktion.

Zunächst werden für mehrere technische Parameter des Analysegerätes die entsprechenden Parameterwerte ermittelt, beispielsweise ein Luftfeuchtigkeits-Wert durch einen Luftfeuchtigkeits-Sensor innerhalb des Analysegerätes, ein Leistungsaufnahme-Wert einer fotometrischen Strahlungsquelle, ein Positions-Wert eines Wischers, ein Zähler-Wert eines Wischeroszillationen-Zählers etc.

Anschließend wird für jeden ermittelten Parameterwert ein relativer oder absoluter Abweichungswert in Bezug auf einen zugeordneten Parameter-Referenzwert ermittelt. Der Referenzwert ist beispielsweise ein Idealwert für den betreffenden Parameter. Der Abweichungswert gibt eine absolute oder relative Abweichung des Parameterwertes von dem Referenzwert an. Der Abweichungswert gibt noch keine unmittelbare Auskunft darüber, wie relevant die Abweichung vom Idealwert für die Funktionalität des Analysegerätes oder für die Messwertqualität ist.

Nach der Ermittlung des Abweichungswertes für alle relevanten Parameter wird ein Abweichungsrelevanz-Wert für jeden Parameter jeweils aus einer parameterspezifischen Funktion ermittelt. Für mindestens zwei Parameter gilt, dass für jeden dieser zwei Parameter die Funktionen voneinander verschieden sind. Durch diese Funktion wird der Zusammenhang zwischen dem Abweichungswert und seiner Relevanz in Bezug auf die Funktionalität des Analysegerätes oder auf die Messwertqualität hergestellt. So kann beispielsweise eine geringe Abweichung des Luftfeuchtigkeits-Wertes von einem Idealwert in einem Gehäuse des Analysegerätes einen Abweichungsrelevanz-Wert von 1,0 generieren, wohingegen eine erheblich größere Abweichung plötzlich zu einem Abweichungsrelevanz-Wert von 0,3 führen kann. Ähnlich kann es sich beispielsweise mit der Leistungsaufnahme einer helligkeitsgesteuerten fotometrischen Lichtquelle verhalten, die erst ab Erreichen eines Schwellenwertes zu einer dramatischen Veränderung des Abweichungsrelevanz- Wertes führt. Bei einem Wischer, der beispielsweise vor jeder Analyse ein Messfenster von Rückständen freiwischen soll, kann die Blockade einer einzigen Wischoszillation beispielsweise zu einem unveränderten Abweichungsrelevanz-Wert von 1,0 führen, wenn unmittelbar nach der Blockade der Wischversuch erfolgreich wiederholt werden kann. Wenn auch bei der Wiederholung eine Blockade auftritt, kann der Abweichungsrelevanz-Wert beispielsweise auf 0,8 verringert werden. In den beschriebenen Beispielen kennzeichnet ein Abweichungsrelevanz-Wert von 1,0 einen optimalen Zustand, und ein Wert von 0,0 einen sehr schlechten Zustand.

Schließlich wird aus den Abweichungsrelevanz-Werten der relevanten Parameter mit Hilfe einer Indikatorfunktion ein Zustandsindikator ermittelt, der beispielsweise ein Gerätezustands-Indikator oder ein Messwertqualitäts-Indikator sein kann. Die Indikatorfunktion für die Ermittlung des Gerätezustands-Indikators kann sich beispielsweise bezüglich der Bedeutung des Parameters "Luftfeuchtigkeit" erheblich von der Indikatorfunktion für die Ermittlung des Messwertqualitäts-Indikators unterscheiden. So kann beispielsweise eine hohe Luftfeuchtigkeit in dem Gehäuse des Analysegerätes für den Gerätezustands-Indikator eine große Bedeutung haben, da sie auf eine Undichtigkeit des Gehäuses hindeutet, während sie für den Messwertqualitäts-Indikator nur von geringer Bedeutung ist, da sie auf die Vertrauenswürdigkeit eines Messwertes keinen unmittelbaren Einfluss hat.

Mit Hilfe des beschriebenen Verfahrens kann ein Zustandsindikator für das Analysegerät generiert werden, der eine aussagekräftige Information über einen bestimmten Aspekt des Messgerätes darstellt. Mit diesem Zustandsindikator bekommen gerade ungeschulte und unerfahrene Anwender eine Information zu einem bestimmten Aspekt des Analysegerätes, ohne hierfür über komplexes Expertenwissen verfügen zu müssen .

Vorzugsweise enthält die Indikatorfunktion einen Term für das arithmetische Mittel mehrerer Abweichungsrelevanz-Werte. Besonders bevorzugt enthält die Indikatorfunktion einen Term mit einer Multiplikation des kleinsten aller Abweichungsrelevanz-Werte mit dem arithmetischen Mittel aller übrigen Abweichungsrelevanz-Werte.

Gemäß einer bevorzugten Ausgestaltung wird ein Parameter durch einen Reagenzmengen-Sensor in einem Vorratstank, einen Feuchtigkeits-Sensor in dem Gerätegehäuse, einen Motorstrom-Sensor eines Antriebsmotors, einen Leistungssensor einer Fotometer-Lichtquelle, einen Wischerblatt-Oszillationszähler, einen Wischerblatt-Blockadesensor, einen Wischerblatt-Positionssensor und/oder einen Antriebsmotor-Rotationszähler ermittelt.

Im Folgenden wird ein Ausführungsbeispiel der Erfindung anhand der Zeichnung näher erläutert.

Die Figur zeigt schematisch ein Wasser-Analysegerät, in dem ein Verfahren zur Bestimmung eines Zustandsindikators verwirklicht ist.

Das in der Figur dargestellte Wasser-Analysegerät 10 ist vorliegend ein ProzessAnalysegerät, das als Tauchsonde ausgebildet ist. Das Analysegerät 10 weist eine Sensoreinheit 12 auf, in der mehrere Sensoren S1, S2, S3 angeordnet sind.

Der Sensor S1 ist ein Feuchtigkeitssensor in dem Gehäuse der Sensoreinheit 12, der die Luftfeuchtigkeit innerhalb des im Betrieb in Wasser eingetauchten Sensoreinheit- Gehäuses misst. Überschreitet die Luftfeuchtigkeit innerhalb des Gehäuses einen bestimmten Grenzwert, kann von einer Undichtigkeit des Gehäuses ausgegangen werden, die dauerhaft zu einer erheblichen Störung und einer Beschädigung der Sensoreinheit 12 beziehungsweise des Analysegerätes 10 führen kann. Die durch den Feuchtigkeitssensor bestimmte Luftfeuchtigkeit ist daher ein Parameter, der im Wesentlichen in eine Aussage über den Gerätezustand eingeht.

Der Sensor S2 ist ein Wischer-Positionssensor, der die Wischerposition eines ein Sensorfenster der Sensoreinheit 12 wischenden Wischers angibt. Der Wischer-Positionssensor kann entweder die exakte Position des Wischers oder aber ein Vorhandensein des Wischers an einer bestimmten Position feststellen. Mit Hilfe des Wischer-Positionssensors kann festgestellt werden, ob der Wischer blockiert, und kann in Verbindung mit einem entsprechenden Zähler die Anzahl von Wisch-Oszillationen gezählt werden. Da das wirkungsvolle Wischen des Messfensters durch den Wischer die Qualität des Messergebnisses beeinflusst, sind Informationen über eine Wischer-Blockade oder den Wischer-Verschleiß relevant für eine Gesamtaussage über die Messwertqualität.

Der Sensor S3 ist ein Lichtquellen-Leistungssensor, der den elektrischen Leistungsbedarf einer helligkeitsgeregelten Fotometer-Lichtquelle angibt. Jede Lichtquelle, auch eine Leuchtdiode, altert durch ihren Betrieb. Das Alter äußert sich häufig insbesondere dadurch, dass für eine konstante Lichtleistung im Alter eine höhere elektrische Leistung erforderlich ist, als für die junge Lichtquelle erforderlich war. Mit der Veränderung des Leistungsbedarfs kann sich ferner auch das emittierte Spektrum der Lichtquelle verändern, was wiederum die Messwertqualität verschlechtern kann. Die von dem Lichtquellen-Leistungssensor gelieferte Information geht also insbesondere in den Gerätezustand ein, kann jedoch auch in die Messwertqualität eingehen.

Als weitere Sensoren können Reagenzmengen-Sensoren vorgesehen sein, die den Füllstand der betreffenden Reagenzien in entsprechende Vorratstanks ermitteln. Ferner kann an dem Antriebsmotor des Wischers ein Motorumdrehungs-Zählersensor vorgesehen sein, mit dem indirekt der Verschleiß des Motors gemessen wird. Mit einem Motorstrom-Sensor können weitere Informationen über den Antriebsmotor und/oder den Wischer gewonnen werden. Der Motorstrom-Sensor kann auch als Wischerblockade-Sensor dienen, mit dem eine Blockade des Wischers festgestellt werden kann. Grundsätzlich können die Informationen jeder Art von Sensoren des Analysegerätes in die Ermittlung eines Messwertqualitäts-Indikators und/oder eines Gerätezustands-Indikators eingehen.

Die von den Sensoren $S_1$, $S_2$, $S_3$ ermittelten Parameterwerte $p_{S1}$, $p_{S2}$, $p_{S3}$ werden einem Mikroprozessor 16 zugeführt, und in Rechenmodulen $D_1$, $D_2$, $D_3$ mit einem Parameter-Referenzwert abgeglichen. Das Abgleichen erfolgt durch eine Differenzbildung oder eine Division mit dem jeweiligen Parameter-Referenzwert. Auf diese Weise wird für jeden Sensor $S_1$, $S_2$, $S_3$ beziehungsweise jeden Parameter ein Abweichungswert $d_{S1}$, $d_{S2}$, $d_{S3}$ ermittelt, der jeweils einer zweiten Stufe von Rechenmodulen 18 zugeführt wird.

Am Beispiel eines einen Relativ-Luftfeuchtigkeits-Wert liefernden Luftfeuchtigkeits-Sensors kann der Referenzwert beispielsweise ein Idealwert von 0% Luftfeuchtigkeit sein. Die Ermittlung eines Abweichungswertes kann durch Differenzbildung oder durch Division erfolgen. Beispielsweise kann der Abweichungswert aus dem Quotienten der Differenz aus dem Parameterwert und dem Referenzwert im Zähler und dem Referenzwert im Nenner bestimmt werden.

Beim Beispiel eines eine Information über Wischer-Blockaden liefernden Wischer-Positionssensors kann der Referenzwert beispielsweise 4 sein. Der Abweichungswert kann sich dann aus der Anzahl der tatsächlichen Wischzyklen bezogen auf die 4 letzten Wischzyklusversuche ergeben, und auf diese Weise einen Wert zwischen 4/4 und 0/4 ergeben.

In den zweiten Rechenmodulen 18 wird aus dem Abweichungswerten $d_{S1}$, $d_{S2}$, $d_{S3}$ mit Hilfe einer parameterspezifischen Abweichungsrelevanz-Funktion $f_{S1}$, $f_{S2}$, $f_{S3}$ für jeden Parameter ein Abweichungsrelevanz-Wert $y_{S1}$, $y_{S2}$, $y_{S3}$ ermittelt. Durch die Abweichungsrelevanz-Funktion werden insbesondere kleine, nicht- bedeutende Abweichungen von einem Idealwert beziehungsweise große Abweichungen von einem Grenzwert als sehr wenig relevant bewertet. Die Abweichungsrelevanz-Funktion ist in aller Regel für jeden Parameter verschieden, und kann beispielsweise in einem Intervall von 0,0 bis 1,0 den Abweichungsrelevanz-Wert angeben, wobei ein Wert von 1,0 den Idealzustand und 0,0 einen sehr schlechten Zustand kennzeichnen.

Insbesondere in den Rechenmodulen 18 wird der Zustandsindikator I individualisiert. Wenn mehrere verschiedene Zustandsindikatoren I für dasselbe Analysegerät 10 bestimmt werden sollen, teilt sich der Ablauf vor den Rechenmodulen 18 in zwei oder mehr Zweige auf, in denen sich insbesondere die jeweiligen Abweichungsrelevanz-Funktionen $f_{s1}$, $f_{s2}$, $f_{s3}$ voneinander unterscheiden.

Die Abweichungsrelevanz-Werte $y_{S1}$, $y_{S2}$, $y_{S3}$ werden alle einem dritten Rechenmodul 20 zugestellt, in dem mit Hilfe einer Indikatorfunktion $f_I$ aus den Abweichungsrelevanz-Werten ein Zustandsindikator I errechnet wird.

Die Indikatorfunktion $f_I$ kann beispielsweise ein Polynom sein, in dem die Abweichungsrelevanz-Werte mit verschieden großen Faktoren versehen sind. Als Zustandsindikator kann beispielsweise ein Gerätezustands-Indikator oder ein Messwertqualitäts-Indikator generiert werden. Während in die Indikatorfunktion für den Gerätezustand beispielsweise die Luftfeuchtigkeit und die Anzahl der Motorumdrehungen in hohem Maße eingehen, können diese in der Indikatorfunktion für die Messwertqualität relativ unbeachtet bleiben.

Bevorzugt besteht die Indikatorfunktion $f_I$ aus einer Multiplikation des kleinsten Abweichungsrelevanz-Wertes $y_{S1}$, $y_{S2}$, $y_{S3}$ mit dem arithmetischen Mittel der übrigen Abweichungsrelevanz-Werte $y_{S1}$, $y_{S2}$, $y_{S3}$.

Der Zustandsindikator I kann anschließend an eine Anzeige 14 oder an einen den Zustandsindikator I weiterverarbeitenden Rechner ausgegeben werden. Der Rechner kann gegebenenfalls entsprechende Eingriffe in das Analysegerät vornehmen, wenn der Zustandsindikator I einen Grenzwert überschreitet.

Die Indikatorfunktion kann beispielsweise folgende Form haben:

$$I = \min f_{si} \times (\textstyle\sum f_{si} - \min f_{si}) / (\max (i) - 1)$$

wobei $f_{si}$ die jeweilige Abweichungsrelevanz-Funktion $f_{s1}$ - $f_{s3}$ ist.

Eine Abweichungsrelevanz-Funktion $f_{si}$ kann beispielsweise die folgende Form haben:

$$f_{si} = (1 - start) \times (1 - d_{si})^n + start,$$

wobei $d_{si}$ ein Abweichungswert $d_{s1}$, $d_{s2}$ oder $d_{s3}$ ist, und die Werte "start" und n die Form der Kurve bestimmen.

**Patentansprüche**

1. Verfahren zur Bestimmung eines Zustandsindikators eines Wasser-Analysegerätes (10), mit den Verfahrensschritten:

   - Ermittlung jeweils eines Parameterwertes ($p_{S1}$, $p_{S2}$, $p_{S3}$) für mindestens zwei verschiedene technische Parameter des Wasser-Analysegerätes (10),
   - Ermittlung eines Abweichungswertes ($d_{S1}$, $d_{S2}$, $d_{S3}$) des Parameterwertes $p_{S1}$, $p_{S2}$, $p_{S3}$ in Bezug auf einen zugeordneten Parameter-Referenzwert für jeweils alle Parameter,
   - Ermittlung jeweils eines Abweichungsrelevanz-Wertes ($y_{S1}$, $y_{S2}$, $y_{S3}$) aus dem Abweichungswert ($d_{S1}$, $d_{S2}$, $d_{S3}$) mit Hilfe einer parameterspezifischen Abweichungsrelevanz-Funktion ($f_{S1}$, $f_{S2}$, $f_{S3}$) für jeweils alle Parameter, wobei sich die Funktionen ($f_{S1}$, $f_{S2}$, $f_{S3}$) voneinander unterscheiden, und
   - Errechnen des Zustandsindikators (I) aus allen ermittelten Abweichungsrelevanz-Werten mit Hilfe einer Indikatorfunktion ($f_I$).

2. Verfahren zur Bestimmung eines Zustandsindikators eines Wasser-Analysegerätes (10) nach Anspruch 1, wobei die Indikatorfunktion ($f_I$) einen Term für das arithmetische Mittel mehrerer Abweichungsrelevanz-Werte ($y_{S1}$, $y_{S2}$, $y_{S3}$) enthält.

3. Verfahren zur Bestimmung eines Zustandsindikators eines Wasser-Analysegerätes (10) nach Anspruch 2, wobei der Term eine Multiplikation des kleinsten aller Abweichungsrelevanz-Werte ($y_{S1}$, $y_{S2}$, $y_{S3}$) mit dem arithmetischen Mittel aller übrigen Abweichungsrelevanz-Werte ($y_{S1}$, $y_{S2}$, $y_{S3}$) enthält.

4. Verfahren zur Bestimmung eines Zustandsindikators eines Wasser-Analysegerätes (10) nach einem der vorangegangenen Ansprüche, wobei der Zustandsindikator (I) ein Gerätezustands-Indikator ist.

5. Verfahren zur Bestimmung eines Zustandsindikators eines Wasser-Analysegerätes (10) nach einem der vorangegangenen Ansprüche, wobei der Zustandsindikator (I) ein Messwertqualitäts-Indikator ist.

6. Verfahren zur Bestimmung eines Zustandsindikators eines Wasser-Analysegerätes (10) nach einem der vorangegangenen Ansprüche, wobei das Wasser-Analysegerät (10) ein Prozessanalysegerät ist.

7. Verfahren zur Bestimmung eines Zustandsindikators eines Wasser-Analysegerätes (10) nach einem der vorangegangenen Ansprüche, wobei ein Parameter von einem Feuchtigkeitssensor ermittelt wird.

8. Verfahren zur Bestimmung eines Zustandsindikators eines Wasser-Analysegerätes (10) nach einem der vorangegangenen Ansprüche, wobei ein Parameter von einem Reagenzmengen-Sensor ermittelt wird.

9. Verfahren zur Bestimmung eines Zustandsindikators eines Wasser-Analysegerätes (10) nach einem der vorangegangenen Ansprüche, wobei ein Parameter der Motorstrom eines Antriebsmotors ist.

10. Verfahren zur Bestimmung eines Zustandsindikators eines Wasser-Analysegerätes (10) nach Anspruch 9, wobei der Antriebsmotor einen Wischer zum Wischen eines Messfensters antreibt.

11. Verfahren zur Bestimmung eines Zustandsindikators eines Wasser-Analysegerätes (10) nach einem der vorangegangenen Ansprüche, wobei nach dem Errechnen des Zustandsindikators (I) dieser an eine andere Gerätekomponente ausgegeben wird.

**Claims**

1. A method for determining a condition indicator of a water analysis apparatus (10), the method comprising:

   - determining a respective parameter value ($p_{S1}$, $p_{S2}$, $p_{S3}$) for each of at least two different technical parameters of the water analysis apparatus (10);
   - determining a respective deviation value ($d_{S1}$, $d_{S2}$, $d_{S3}$) of each of the parameter values $p_{S1}$, $p_{S2}$, $p_{S3}$ with respect to an associated respective parameter reference value for each of the technical parameters;
   - determining a respective deviation relevance value ($y_{S1}$, $y_{S2}$, $Y_{S3}$) from each of the deviation values ($d_{S1}$, $d_{S2}$, $d_{S3}$) using a respective parameter-specific deviation relevance function ($f_{S1}$, $f_{S2}$, $f_{S3}$) for each of the parameters, the functions ($f_{S1}$, $f_{S2}$, $f_{S3}$) being different from each other; and
   - calculating, using an indicator function ($f_I$), a condition indicator (I) from all of the determined deviation relevance values.

2. The method for determining a condition indicator of a water analysis apparatus (10) as recited in claim 1, wherein the indicator function ($f_I$) includes a term for an arithmetic mean of a plurality of the deviation relevance values ($y_{S1}$, $y_{S2}$, $y_{S3}$).

3. The method for determining a condition indicator of a water analysis apparatus (10) as recited in claim 2, wherein the term includes a multiplication of the smallest of the deviation relevance values ($y_{S1}$, $y_{S2}$, $y_{S3}$) by an arithmetic mean of all other of the deviation relevance values ($y_{S1}$, $y_{S2}$, $y_{S3}$).

4. The method for determining a condition indicator of a water analysis apparatus (10) as recited in one of the preceding claims, wherein the condition indicator (I) is an apparatus condition indicator.

5. The method for determining a condition indicator of a water analysis apparatus (10) as recited in one of the preceding claims, wherein the condition indicator (I) is a measured value quality indicator.

6. The method for determining a condition indicator of a water analysis apparatus (10) as recited in one of the preceding claims, wherein the water analysis apparatus (10) is a process analysis apparatus.

7. The method for determining a condition indicator of a water analysis apparatus (10) as recited in one of the preceding claims, wherein a parameter is determined by a humidity sensor.

8. The method for determining a condition indicator of a water analysis apparatus (10) as recited one of the preceding claims, wherein a parameter is determined by a reagent quantity sensor.

9. The method for determining a condition indicator of a water analysis apparatus (10) as recited in one of the preceding claims, wherein a parameter is the motor current of a drive motor.

10. The method for determining a condition indicator of a water analysis apparatus (10) as recited in claim 9, wherein the drive motor is configured to drive a wiper for wiping a measurement window.

11. The method for determining a condition indicator of a water analysis apparatus (10) as recited in one of the preceding claims, wherein, after it has been calculated, the condition indicator (I) is supplied to another apparatus component.

**Revendications**

1. Procédé de détermination d'un indicateur d'état d'un appareil d'analyse de l'eau (10), le procédé comprenant les étapes suivantes:

- la détermination d'une valeur de paramètre ($p_{S1}$, $p_{S2}$, $P_{S3}$) respectif pour au moins deux paramètres techniques différents de l'appareil d'analyse de l'eau (10);
- la détermination d'une valeur de déviation ($d_{S1}$, $d_{S2}$, $d_{S3}$) de la valeur de paramètre $p_{S1}$, $p_{S2}$, $p_{S3}$ par rapport à une valeur de paramètre de référence pour chaque paramètre;
- la détermination d'une valeur de relevance de la déviation ($y_{S1}$, $y_{S2}$, $y_{S3}$) à partir de la valeur de déviation ($d_{S1}$, $d_{S2}$, $d_{S3}$) à l'aide d'une fonction de relevance de la déviation ($f_{S1}$, $f_{S2}$, $f_{S3}$), spécifique au paramètre, pour chaque paramètre, les fonctions ($f_{S1}$, $f_{S2}$, $f_{S3}$) étant différentes l'une de l'autre; et
- la calculation, à l'aide d'une fonction d'indicateur ($f_I$), de l'indicateur d'état (I) à partir de toutes les valeurs de relevance de la déviation calculées.

**2.** Procédé de détermination d'un indicateur d'état d'un appareil d'analyse de l'eau (10) selon la revendication 1, dans lequel la fonction d'indicateur ($f_I$) comprend un terme pour la moyenne arithmétique de plusieurs valeurs de relevance de la déviation ($y_{S1}$, $y_{S2}$, $y_{S3}$).

**3.** Procédé de détermination d'un indicateur d'état d'un appareil d'analyse de l'eau (10) selon la revendication 2, dans lequel le terme comprend une multiplication de la plus petite de toutes les valeurs de relevance de la déviation ($y_{S1}$, $y_{S2}$, $y_{S3}$) par la moyenne arithmétique de toutes les autres valeurs de relevance de la déviation ($y_{S1}$, $y_{S2}$, $y_{S3}$).

**4.** Procédé de détermination d'un indicateur d'état d'un appareil d'analyse de l'eau (10) selon l'une quelconque des revendications précédentes, dans lequel l'indicateur d'état (I) est un indicateur de l'état de l'appareil.

**5.** Procédé de détermination d'un indicateur d'état d'un appareil d'analyse de l'eau (10) selon l'une quelconque des revendications précédentes, dans lequel l'indicateur d'état (I) est un indicateur de la qualité de la valeur mesurée.

**6.** Procédé de détermination d'un indicateur d'état d'un appareil d'analyse de l'eau (10) selon l'une quelconque des revendications précédentes, dans lequel l'appareil d'analyse de l'eau (10) est un appareil d'analyse de processus.

**7.** Procédé de détermination d'un indicateur d'état d'un appareil d'analyse de l'eau (10) selon l'une quelconque des revendications précédentes, dans lequel un paramètre est déterminé par un capteur d'humidité.

**8.** Procédé de détermination d'un indicateur d'état d'un appareil d'analyse de l'eau (10) selon l'une quelconque des revendications précédentes, dans lequel un paramètre est déterminé par un capteur de quantité de réactant.

**9.** Procédé de détermination d'un indicateur d'état d'un appareil d'analyse de l'eau (10) selon l'une quelconque des revendications précédentes, dans lequel un paramètre est le courant du moteur d'un moteur d'entrainement.

**10.** Procédé de détermination d'un indicateur d'état d'un appareil d'analyse de l'eau (10) selon la revendication 9, dans lequel le moteur d'entrainement entraine un essuie-glace pour essuyer une fenêtre de mesurage.

**11.** Procédé de détermination d'un indicateur d'état d'un appareil d'analyse de l'eau (10) selon l'une quelconque des revendications précédentes, dans lequel, après avoir fait la calculation dudit indicateur d'état (I), celui est fourni à un autre élément de l'appareil.

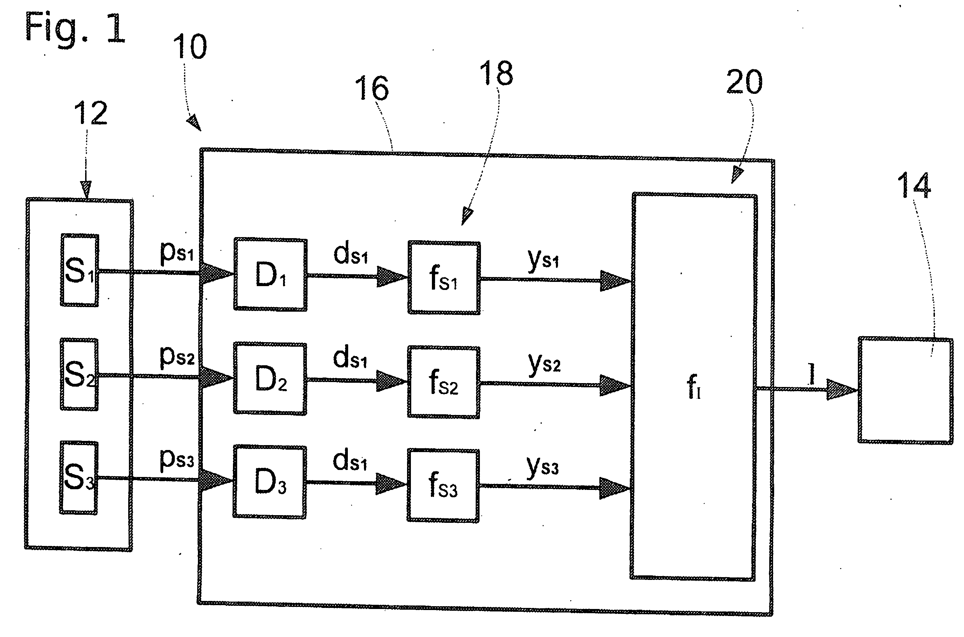

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004063468